# EUROPEAN PATENT APPLICATION

(11) **EP 1 386 580 A1**
(43) Date of publication of application: **04.02.2004**
(21) Application number: 03017303.3
(22) Date of filing: 30.07.2003
(51) Int. Cl.: A61B 5/0492

(54) **Electrode device**

(30) Priority: 31.07.2002 JP 2002223635
(71) Applicant: NTT DoCoMo, Inc., Tokyo 100-6150 (JP)
(72) Inventor: Manabe, Hiroyuki, c/o Intell. Prop. Dept., cho 2-chome, Chiyoda-ku, Tokyo 100-6150 (JP); Hiraiwa, Akira, Deceased (JP); Hiraiwa, Yumiko, Yokohama-shi, Kanagawa 221-0814 (JP)
(74) Representative: Grosse, Wolfgang, Dipl.-Ing.

(57) **Abstract**

The object is to provide an electrode device which can flexibly follow up the movement of the skin in an active electrode device. In an active electrode device, a flexible section, made of a material which is non-conductive and can be transformed flexibly, is disposed between a preamplifier section which is comprised of a hard preamplifier board and a case for protection thereof, and electrode sections made of silver, silver chloride, or the like. By this, the electrode section can move flexibly and can flexibly follow up the movement of the skin.

## Description

### BACKGROUND OF THE INVENTION

### Field of the Invention

The present invention relates to an electrode device for measuring various bio-signals, such as the myoelectrogram of human muscle.

### Related Background of the Invention

Conventional electrode devices for measuring myoelectrogram are classified into passive electrode devices where the electrode section and the amplifier are separate, and active electrode devices where the electrode section and the amplifier are integrated. Passive electrode devices are, for example, aplate electrode, needle electrode and disposable electrode.

Passive electrode devices have been used as conventional electrode devices for medical use and inspection. Passive electrode devices, where the amplifier for amplifying myoelectrogram and the electrode section are separated, has a problem that noise easily enters. Also in order to decrease the contact impedance between the electrode section and skin, paste or electrolytes must be inserted between the electrode section and the skin, which increases handling for measurement. Myoelectrogram have been used mainly for medical and inspection purposes, so measurement is performed in the inspection room of a hospital, where the entry of noise can be decreased by shielding the inspection room, therefore noise was not a major problem. Also the time taken to paste the electrode section to the skin was not a major problem either, since it is medical related personnel who pastes the electrode section on the skin, and it is important to always perform measurement under predetermined conditions.

Currently research on measuring myoelectrogram and using these measurements for engineering is progressing. Examples of such research are: CyberFinger by the present inventor Hiraiwa and others (Hiraiwa, Uchida, Shimohara, Sonehara, "EMG Recognition with a Neural Network Model for Cyber Finger Control", Transactions of the Society of Instrument and Control Engineers, Vol. 30, No. 2, pp. 216 - 224, 1994), and Mime Speech Recognition by the present inventor Manabe and others (Manabe, Hiraiwa, Sugimura, "Speech Recognition with EMG -Vowel Discrimination in a Steady State-", Interaction 2002, pp.181-182, 2002). The problem here is that myoelectrogram must be measured while suppressing the entry of noise even when the measuring location is outside the shielded room. Also when the practical application is considered, taking time to paste the electrode section on the skin is a major problem.

An electrode, which object is to decrease noise, is the above mentioned active electrode device (electrode device where the electrode section and the preamplifier are integrated). If this active electrode device is used, not only is noise decreased but also handling in pasting the electrode section can be minimized, since paste does not have to be inserted between the electrode system and the skin.

However active electrode devices also have a problem. That is, the electrode section is hard because the electrode section and the preamplifier are integrated. In other words, the electrode section, which is hard, cannot follow up the movement of the skin due to the activity of the muscles, so the contact status between the skin and the electrode section changes. In the case of passive electrode devices (particularly a plate electrode and disposable electrode), the electrode can follow up the movement of the skin to a certain extent because paste is or electrolytes are inserted between the electrode section and the skin, and because two electrode sections for measuring myoelectrogram are installed separately.

Whereas in the active electrode device, the electrode section is hard, paste or electrolytes need not be inserted, and two electrode sections are integrated, therefore the electrode section cannot follow up the movement of the skin. For example, when the myoelectrogram of the orbicular muscle of the mouth is measured, the two electrode sections and the skin must always be contacted under predetermined conditions without changing the contact status from the status where no force is applied to the status where the skin is transformed dramatically, as seen when the English "u" sound is vocalized.

With the active electrode device, following up the movement of skin can be improved by inserting paste or electrolytes between the electrode section and the skin, but this negates the advantage of the active electrode device, where handling when the electrode section is pasted to the skin is easy.

Therefore an ideal electrode device for measuring myoelectrogram must meet the following requirements. First, the entry of noise must be low. This can be implemented by integrating the preamplifier into the electrode section, just like the case of the active electrode device. Second is that the electrode must flexibly follow up the movement of the skin due to the activity of the muscles. By implementing both of these aspects, myoelectrogram can be measured in any place and from any region of the body, and the possibility of measuring myoelectrogram and applying them to engineering purposes can be dramatically increased.

With the foregoing in view, it is an object of the present invention to provide an active electrode device which satisfies the first requirement, wherein the second requirement of flexibly following up the movement of the skin can be implemented.

### SUMMARY OF THE INVENTION

To achieve the above mentioned object, the electrode device according to the present invention is, an electrode device for measuring bio-signals of a human, comprising a plurality of electrode sections which are contacted to the skin of a human body for measurement, a preamplifier section which is electrically connected with each electrode section via an electric wire, and a flexible section which is disposed between each electrode section and preamplifier section, and is conductive and can be transformed flexibly.

In the electrode device, a flexible section which is non-conductive and can be transformed flexibly is disposed, so each electrode section can move flexibly according to the movement of the skin. In other words, the present invention makes it possible to flexibly follow up the movement of the skin using the active electrode device. The electrode device according to the present invention cannot only be applied to the measurement of myoelectrogram, but also to measuring other bio-signals, such as brain waves and electro-cardiograms.

The electrode device is also, an electronic device for measuring bio-signals of a human, comprising a plurality of electrode sections which are contacted to the skin of a human body for measurement, and a preamplifier section which is electrically connected with each electrode section via an electric wire, and is made of flexible material which can be transformed flexibly.

In the electrode device, the preamplifier section, which is electrically connected with each electrode section via an electric wire, is made of a flexible material which can be transformed flexibly, and each electrode section can move flexibly according to the movement of the skin.

The electrode device according to the present invention is also an electrode device for measuring bio-signals of a human, comprising: a plurality of electrode sections which are connected to the skin of a human body for measurement, and a flexible section which contains a preamplifier section electrically connected with each electrode section via an electric wire, and is non-conductive and can be transformed flexibly.

In the electrode device, the flexible section contains the preamplifier section electrically connected with each electrode section via an electric wire, and is non-conductive and can be transformed flexibly, so each electrode section can move flexibly according to the movement of the skin.

To measure myoelectrogram, generally it is necessary to measure the potential difference between two electrode sections. Therefore when two electrode sections are disposed, only one type of myoelectrogram can be detected. However if three or more electrode sections are disposed, the types of myoelectrogram for the number of combinations when two out of three or more electrode sections are selected, that is, three or more types of myoelectrogram, can be detected. Therefore more types of myoelectrogram than the normal case of two electrode sections can be detected, and efficient detection processing and an improvement in detection accuracy can be implemented.

If only the flexible section or both the flexible section and the preamplifier section are shielded with a conductive material, the entry of noise between the electrode section and the preamplifier section can be prevented.

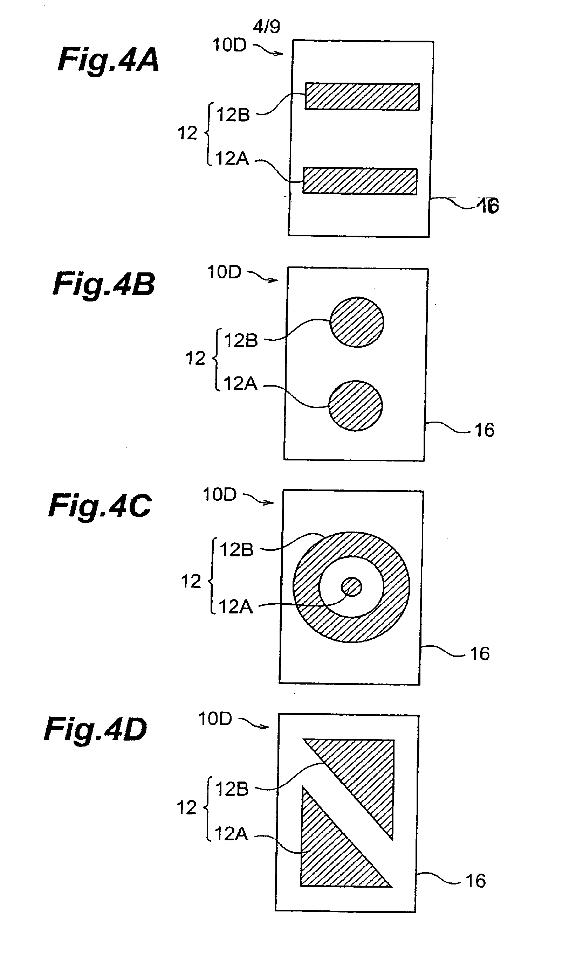

If the shape or the size of the electrode section is set according to the measurement target region, a more efficient measurement can be performed. For example, compared with the bar type electrode section 12 in Fig. 4A, the circular electrode section 12 in Fig. 4B has a wide contact area with the skin, so contact resistance can be decreased. However the circular electrode section 12 in Fig. 4B tends to cause the entry of unnecessary myoelectrogram (cross-talk) since the contact area with the skin is wide. The concentric type electrode section 12 in Fig. 4C allows measurement of myoelectrogram at a pin point, since the two electrode sections can be put together in a narrow area. The triangular electrode section 12 in Fig. 4D allows taking a large contact area with skin.

If the flexible section or the flexible material is further comprised of a plurality of layers, and the elastic coefficient of each layer is set according to the movement of the measurement target region, the electrode device can operate following up the movement of the skin due to the activity of muscles more closely.

If the elastic coefficient in the flexible section or the flexible material is set so as to continuously change according to the movement of the measurement target region, the electrode device can operate with following up with the movement of the skin due to the activity of the muscles more closely.

If a step difference is created between the plurality of electrode sections according to the measurement target region, contact between each electrode section and the skin can be further improved.

### BRIEF DESCRIPTION OF THE DRAWINGS

Fig. 1 is a structural diagram depicting the principle of the present invention and the first embodiment.

Fig. 2 is a structural diagram depicting the electrode of the second embodiment.

Fig. 3 is a structural diagram depicting the electrode of the third embodiment.

Fig. 4A is a structural diagram depicting an example of a bar type electrode of the fourth embodiment.

Fig. 4B is a structural diagram depicting an example of a circular electrode of the fourth embodiment.

Fig. 4C is a structural diagram depicting an example of a concentric electrode of the fourth embodiment.

Fig. 4D is a structural diagram depicting an example of a triangular electrode of the fourth embodiment.

Fig. 5A is a structural diagram depicting an example of the electrode when a metal wire is used for wiring the electrode and the preamplifier of the fifth embodiment.

Fig. 5B is a structural diagram depicting an example of the electrode when a non-metal conductor is used for wiring the electrode and the preamplifier of the fifth embodiment.

Fig. 6 is a structural diagram depicting the electrode of the sixth embodiment;

Fig. 7 is a structural diagram depicting the electrode of the seventh embodiment;

Fig. 8 is a structural diagram depicting the electrode of the eighth embodiment; and

Fig. 9 is a structural diagram depicting the electrode of the ninth embodiment.

### DESCRIPTION OF THE PREFERRED EMBODIMENTS

Embodiments of the electrode devices according to the present invention will now be described in sequence.

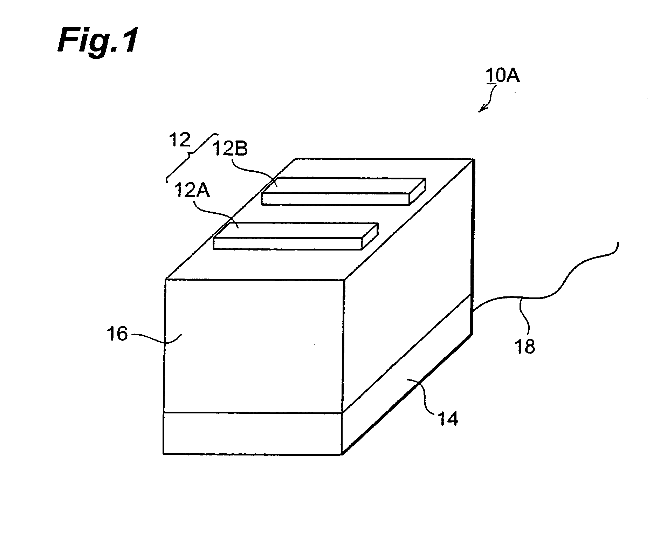

Fig. 1 shows a configuration example of the electrode device 10A according to the first embodiment. Fig. 1 shows the embodiment when two electrode sections, 12A and 12B, are disposed.

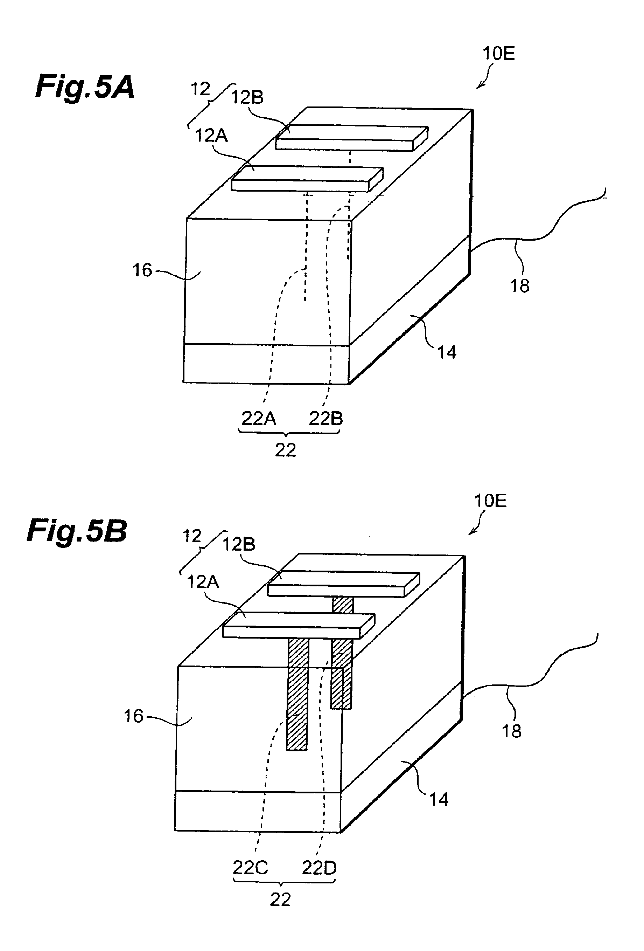

This electrode device 10A is a so-called active electrode, and comprises an electrode section 12, a flexible section 16, a preamplifier section 14 and a cable for external connection 18. The preamplifier section 14 further comprises a hard preamplifier base and a case for protecting the base. The flexible section 16 is made of material which is non-conductive and transformed flexibly, such as silicon and non-conductive rubber. The electrode section 12 further comprises the electrode sections 12A and 12B made of silver or silver chloride, for example. Each one of the electrode sections 12A and 12B and the preamplifier section 14 are wired by a lead wire (described later with reference to Figs. 5A, 5B), which is wired inside the flexible section 16. By the cable for external connection 18, power is supplied to the preamplifier section 14 and can be output from the preamplifier section 14.

In this way, according to the electrode device 10A of the first embodiment, in the active electrode where the preamplifier section 14 and the electrode section 12 are separated, the flexible section 16, made of a material which is non-conductive and transformed flexibly, is disposed between the preamplifier section 14 and the electrode section 12, so the electrode section 12 can move flexibly and can follow up the movement of skin flexibly.

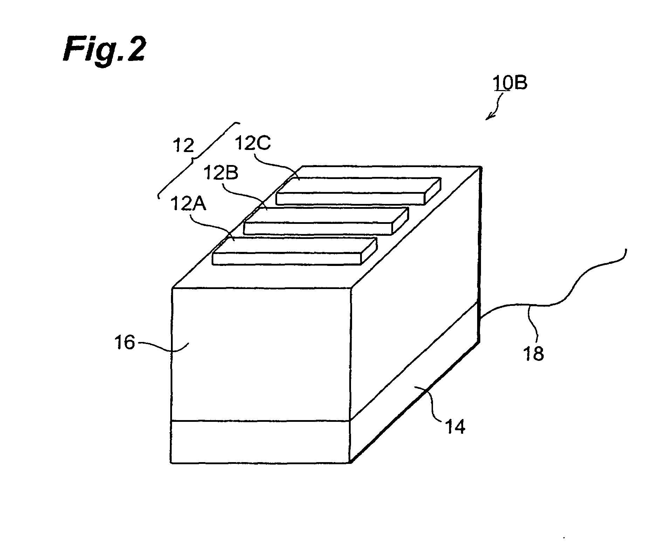

Fig. 2 shows a configuration example of the electrode device 10B according to the second embodiment. Fig. 2 shows the embodiment when three electrode sections, 12A, 12B and 12C, are disposed. Generally it is necessary to measure the potential difference between the two electrode sections to measure myoelectrogram. Therefore when two electrode sections are disposed, only one type of myoelectrogram can be detected. Whereas in the electrode device 10B shown in Fig. 2, three electrode sections are disposed, so the types of myoelectrogram for the number of combinations when two out of three electrode sections are selected, that is, three types of myoelectrogram, can be detected.

In this way, in the case of the electrode device 10B of the second embodiment, three electrode sections are disposed and three types of myoelectrogram can be detected, therefore more types of myoelectrogram, than the normal case of two electrode sections, can be detected, and efficient detection processing and an improvement of detection accuracy can be implemented.

Other than the case of the three electrode sections shown in Fig. 2, the number of types of detectable myoelectrogram can be increased by increasing the number of electrodes to be 4 or 5.

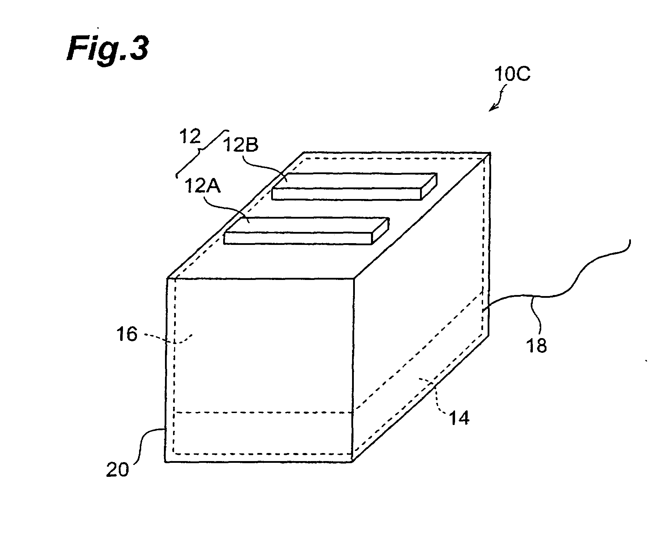

Fig. 3 shows a configuration example of the electrode device 10C according to the third embodiment. In the embodiment shown in Fig. 3, the outside of the flexible section 16 and the preamplifier section 14 shown in Fig. 1 is covered with a conductive flexible material 20, such as conductive rubber, so as to shield the entire electrode device 10C.

In the active electrode device to which the present invention is applied, noise may enter between the electrode section and the preamplifier section, since the electrode section and the preamplifier section are somewhat apart. Whereas in the present embodiment, the entry of noise is prevented by shielding the area between the electrode section 12 and the preamplifier section 14. In other words, as Fig. 3 shows, the outside of the preamplifier section 14 and the flexible section 16 is covered with a conductive and flexible material 20, which makes it possible to shield the area between the electrode section 12 and the preamplifier section 14.

Design must be such that the electrode section 12 does not contact with the conductive material 20. If the preamplifier section 14 need not be shielded, then the conductive material 20 may shield only the flexible section 16. Since the preamplifier section 14 is hard, the material to cover the preamplifier section 14 need not be a material which can be transformed flexibly.

Figs. 4A - 4D show configuration examples of the electrode device 10D according to the fourth embodiment. As Figs. 4A - 4D show, various shapes can be used as the shape of the electrode section 12. For example, Fig. 4A shows a bar type electrode section 12, Fig. 4B shows a circular electrode section 12, Fig. 4C shows a concentric electrode section 12, and Fig. 4D shows a triangular electrode section 12.

Also various sizes can be used for the size of the electrode sections 12. A combination of the electrode sections in Figs. 4A - 4D may be used.

By selecting and using these shapes of the electrode section 12 according to the muscle to be measured, a more efficient measurement can be performed. For example, compared with the bar type electrode section 12 in Fig. 4A, the circular electrode section 12 in Fig. 4B has a wider contact area with the skin, so contact resistance can be decreased. However, the circular electrode section 12 in Fig. 4B tends to cause the entry of unnecessary myoelectrogram (cross-talk) since the contact area with the skin is wide.

The concentric type electrode section 12 in Fig. 4C allows the measurement of myoelectrogram at a pin point, since two electrode sections can be put together in a narrow area. The triangular electrode section 12 in Fig. 4D allows taking a large contact area with the skin.

If the above mentioned various shapes and sizes of the electrode section are selected and used according to the muscle to be measured, an even more efficient measurement can be performed.

Figs. 5A, 5B show configuration examples of the electrode device 10E according to the fifth embodiment. In the fifth embodiment, the wiring of the electrode section 12 and the preamplifier section 14 will be described with reference to Figs. 5A, 5B. For the wiring of the electrode section 12 and the preamplifier section 14, a simple metal wire, such as the lead wire 22 shown in Fig. 5A, can be used. In other words, the lead wire 22A wires the electrode section 12A and the preamplifier section 14, and the lead wire 22B wires the electrode section 12B and the preamplifier section 14. These lead wires 22 need to be transformed flexibly according to the transformation of the flexible section 16.

For the wiring of the electrode section 12 and the preamplifier section 14, a non-metal conductor, such as the conductive rubber shown in Fig. 5B, may be used. In other words, the lead wire 22c, which is a non-metal conductor, wires the electrode section 12A and the preamplifier section 14, and the lead wire 22D, which is a non-metal conductor, wires the electrode section 12B and the preamplifier section 14. In this case as well, these lead wires 22 need to be transformed flexibly according to the transformation of the flexible section 16.

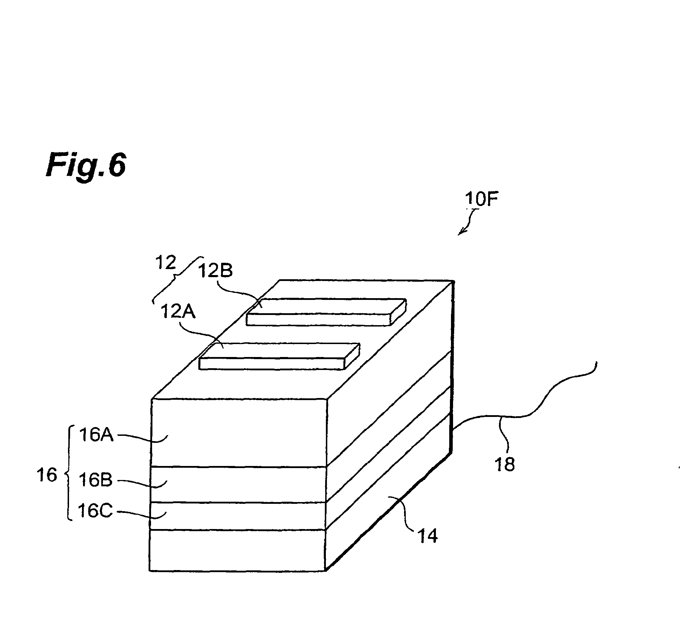

Fig. 6 shows a configuration example of the electrode device 10F according to the sixth embodiment. In this electrode device 10F, the flexible section 16 is divided into three layers, and is comprised of the flexible section 16A, flexible section 16B and flexible section 16C from the electrode section 12 side. Each flexible section has a separate elastic coefficient, and is designed, for example, such that the flexible section 16c at the preamplifier side 14 is the hardest, and the flexible section 16A at the electrode section 12 side is the most flexible. By changing the elastic coefficient in steps in the flexible section 16 in this way, the electrode device can operate with following up with the movement of the skin due to the activity of the muscles more closely.

In the flexible section 16, the elastic coefficient may be changed in steps as shown in Fig. 6, or may be changed continuously. If the elastic coefficient is changed in steps, the layer structure need not be a three layer structure, but may be two layer, or a four or more layer structure. It is not necessary that the layer at the preamplifier section 14 side is the hardest, and the layer at the electrode section 12 side is the most flexible, but setting an appropriate elastic coefficient according to the movement of the skin area to be measured is preferable.

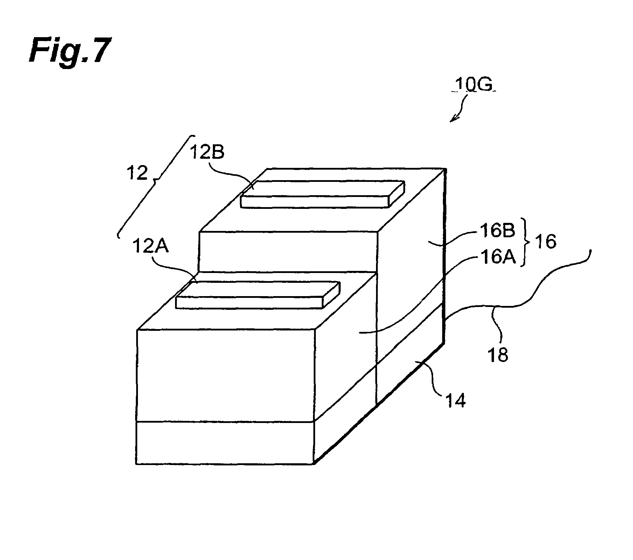

Fig. 7 shows a configuration example of the electrode device 10G according to the seventh embodiment. In this electrode device 10G, a step difference is created between the electrode sections 12A and 12B, by creating a difference in height (dimension in the vertical direction in Fig. 7) between the flexible section 16A where the electrode section 12A is placed and the flexible section 16B where the electrode section 12B is placed.

By creating a step difference in the electrode section 12 in this way, contact between the electrode section 12 and the skin can be further increased. The size of the step difference must be set according to the shape of the muscle and the skin to be measured. A configuration where the height in the electrode sections 12A and 12B is continuously changed, rather than creating a step difference as in Fig. 7, may be used.

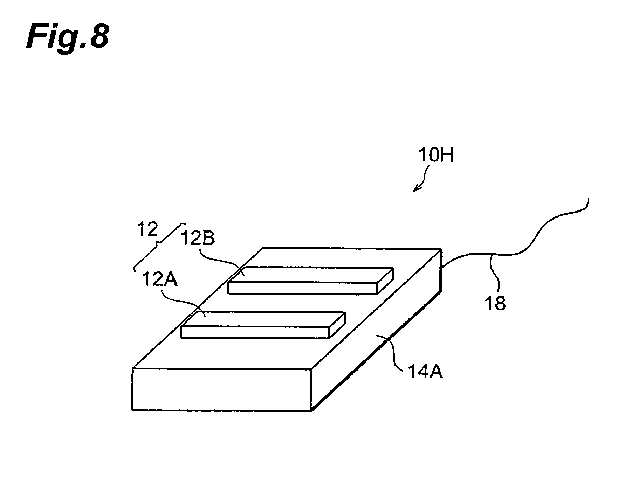

Fig. 8 shows a configuration example of the electrode device 10H according to the eighth embodiment. This electrode device 10H is characterized in that the preamplifier section 14A is made of a flexible material. In other words, the preamplifier with 14A itself can be flexible by using a flexible board which is commercially available. By this, the entire size of the electrode device 10H can be decreased.

The flexible preamplifier section 14A shown in Fig. 8 can certainly be used for the preamplifier sections in Fig. 1 - Fig. 7. In this case, an electrode device which is more flexible and which can be decreased in size can be implemented.

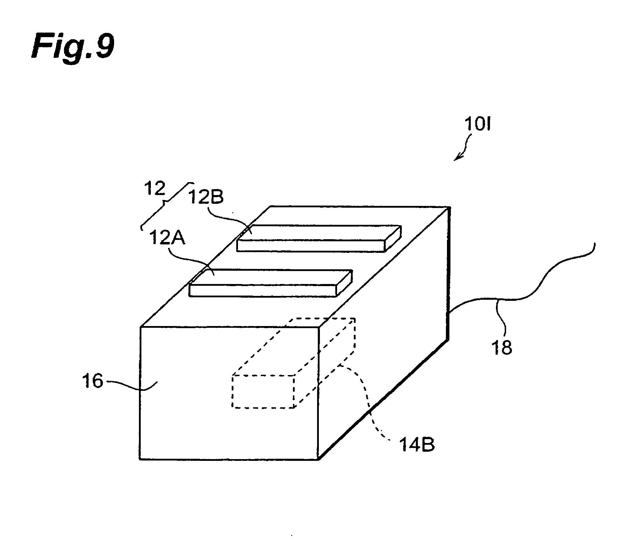

Fig. 9 shows a configuration example of the electrode device 10I according to the ninth embodiment. This electrode device 10I is characterized in that the preamplifier section 14B is contained in the flexible section 16. By this configuration, the preamplifier section 14B, which is an electronic circuit component, can be sufficiently protected from external shock. Also the configuration shown in Fig. 9 makes the external case, required for the preamplifier section in Fig. 1 - Fig. 8, unnecessary, so further downsizing and decreasing the cost of the entire electrode device can be implemented.

The electrode devices in Fig. 1 - Fig. 9 cannot only be applied to the measurement of myoelectrogram of a human body, but also can be applied to measuring other bio-signals, such as brain waves and electro cardiograms.

As described above, according to the present invention, each electrode section can move flexibly according to the movement of the skin. In other words, the shortcoming of using an active electrode, that is where following up the movement of the skin flexibly is difficult, can be solved.

## Claims

1. An electrode device for measuring bio-signals of a human, comprising:
a plurality of electrode sections which are contacted to the skin of a human body for measurement;
a preamplifier section which is electrically connected with each electrode section via an electric wire; and
a flexible section which is disposed between each electrode section and the preamplifier section, and is non-conductive and can be transformed flexibly.

2. An electrode device for measuring bio-signals of a human, comprising:
a plurality of electrode sections which are contacted to the skin of a human body for measurement; and
a preamplifier section which is electrically connected with each electrode section via an electric wire and is made of flexible material which can be transformed flexibly.

3. An electrode device for measuring bio-signals of a human, comprising:
a plurality of electrode sections which are contacted to the skin of a human body for measurement; and
a flexible section which contains a preamplifier section electrically connected with each electrode section via an electric wire, and is non-conductive and can be transformed flexibly.

4. The electrode device according to Claim 1, wherein the shape or size of said electrode section is set according to the measurement target region.

5. The electrode device according to Claim 1, wherein said flexible section or said flexible material is comprised of a plurality of layers, and the elastic coefficient of each layer is set according to the movement of the measurement target region.

6. The electrode device according to Claim 1, wherein the elastic coefficient in said flexible section or said flexible material is set so as to continuously change according to the movement of the measurement target region.
